# EUROPEAN PATENT APPLICATION

(11) **EP 4 684 739 A1**
(43) Date of publication of application: **28.01.2026**
(21) Application number: 24810879.7
(22) Date of filing: 07.05.2024
(51) Int. Cl.: A61B 8/12

(54) **ULTRASONIC INSPECTION DEVICE AND INTRAVASCULAR ULTRASONIC INSPECTION CATHETER**

(30) Priority: 22.05.2023 JP 2023084171
(71) Applicant: Nipro Corporation, Settsu-shi, Osaka 566-8510 (JP)
(72) Inventor: MIYAGAWA, Katsuya, Settsu-shi, Osaka 566-8510 (JP); MATSUMOTO, Misa, Settsu-shi, Osaka 566-8510 (JP)
(74) Representative: Kraus & Lederer PartGmbB
(86) International application number: PCT/JP2024/017014
(87) International publication number: WO 2024/241871

(57) **Abstract**

[Problem] To provide an ultrasound examination device that has a small outer diameter and is electrically stable.

[Solution] An ultrasound examination device 14 includes a shaft 21, a first lead wire 22A and a second lead wire 22B inserted into an internal space of the shaft 21 and extended out from a distal end of the shaft 21, a conductive thin film 23 extending from the distal end of the shaft 21 toward a distal side and electrically connected to the first lead wire 22A, an ultrasound probe 25 located on the distal side of the distal end of the shaft 21 and having a first electrode layer 52 electrically connected to the conductive thin film 23 and a second electrode layer 54 electrically connected to the second lead wire 22B, and a resin holder 26 configured to hold, at least, a portion belonging to the second lead wire 22B and extended out from the shaft 21, the conductive thin film 23, and the ultrasound probe 25.

## Description

### TECHNICAL FIELD

The present invention relates to an ultrasound examination device suitable for inserting into a blood vessel and a catheter for an intravascular ultrasound examination.

### BACKGROUND ART

Conventionally, before performing a treatment of removing an embolism that occurs in a blood vessel by inserting a catheter into the blood vessel and excising the embolism, obtaining an ultrasound image of the blood vessel by inserting a catheter having an ultrasound probe into the blood vessel is performed. An outer diameter of an outer shaft into which the ultrasound probe is inserted is equal to less than several millimeters. In view of reducing a burden on a patient, inserting the catheter into a thin blood vessel, or the like, it is desirable that an outer diameter of the catheter be smaller. Furthermore, a catheter having a thin diameter is desirable also for using the catheter in treating occlusive lesions or for cerebral blood vessels. As a method for downsizing the ultrasound probe located in the catheter, a manufacturing method described in Patent Document 1 is devised.

### PRIOR ART DOCUMENT

### PATENT DOCUMENT

[Patent Document 1] Japanese Patent No. 5225670 gazette

### SUMMARY OF THE INVENTION

### PROBLEMS TO BE SOLVED BY THE INVENTION

Lead wires that supply an electric power or transmit electrical signals are connected to the ultrasound probe, however, even if the ultrasound probe is downsized, it is difficult to make the outer diameter of the catheter small unless connection portions to the lead wires are downsized. On the other hand, if the lead wires are made excessively thin or the connection portions are made excessively small, a risk of connection defects such as a breakage of wire or a short circuit increases or manufacturing becomes difficult.

The present invention has been made in view of such circumstances, and an object thereof is to provide an ultrasound examination device that has a small outer diameter and is electrically stable.

### MEANS FOR SOLVING THE PROBLEMS

(1) An ultrasound examination device according to the present invention includes a shaft, a first lead wire and a second lead wire inserted into an internal space of the shaft and extended out from a distal end of the shaft, a conductive thin film extending from the distal end of the shaft toward a distal side and electrically connected to the first lead wire, an ultrasound probe located on the distal side of the distal end of the shaft and having a first electrode layer electrically connected to the conductive thin film and a second electrode layer electrically connected to the second lead wire, and a resin holder configured to hold, at least, a portion belonging to the second lead wire and extended out from the shaft, the conductive thin film, and the ultrasound probe.

Since the first lead wire is electrically connected to the ultrasound probe through the conductive thin film, an outer diameter dimension in a vicinity of the ultrasound probe can be made small.

(2) Preferably, the ultrasound examination device further includes an insulating support member located between the distal end of the shaft and the ultrasound probe so as to be held by the resin holder, and configured to support the second lead wire.

A breakage of the second lead wire near a connection point to the ultrasound probe is suppressed.

(3) Preferably, the ultrasound probe has a flat plate shape in which a backing layer, the first electrode layer, a piezoelectric element sheet, and the second electrode layer are laminated, the conductive thin film is connected to a first surface of the ultrasound probe on a side of the backing layer, and the second lead wire is connected to a second surface on a side of the second electrode.

Since the conductive thin film and the second lead wire do not come close, a short circuit between the conductive thin film and the second lead wire is suppressed.

(4) Preferably, the first lead wire and the second lead wire form a coaxial cable with the second lead wire located at a center.

The internal space of the shaft for inserting the first lead wire and the second lead wire can be made small.

(5) Preferably, the resin holder is configured to internally hold the portion belonging to the second lead wire and extended out from the shaft, the conductive thin film, and the ultrasound probe.

According to the above-described configuration, since the portion belonging to a second lead sensor and extended out from the shaft, the conductive thin film, and the ultrasound probe are insulated by the resin holder, an individual insulation processing for each component is unnecessary.

(6) The present invention may also be directed to a catheter for an intravascular ultrasound examination including the ultrasound examination device, and an outer shaft into which the ultrasound examination device is inserted rotatably taking the shaft as an axis.

### EFFECTS OF THE INVENTION

According to the present invention, an ultrasound examination device that has a small outer diameter and is electrically stable is realized.

### BRIEF DESCRIPTION OF THE DRAWINGS

Fig. 1 is a diagram showing an external configuration of a catheter 10.
Fig. 2 is a schematic diagram showing a vicinity of a distal end of an ultrasound examination device 14.

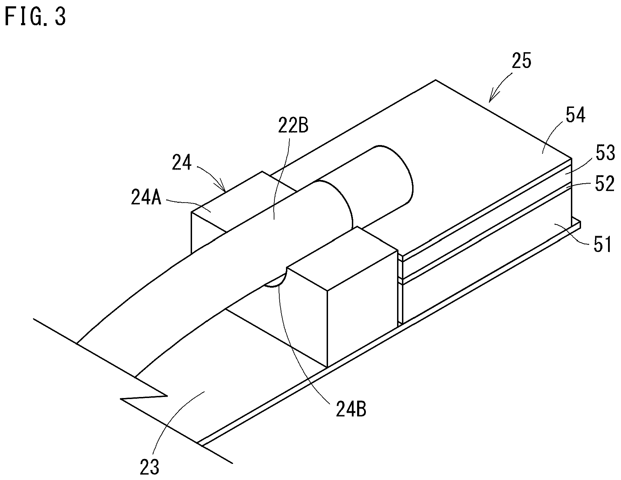

Fig. 3 is a perspective view showing the vicinity of the distal end of the ultrasound examination device 14.
Fig. 4 is a schematic diagram showing a manufacturing method of the ultrasound examination device 14.
Fig. 5 is a schematic diagram showing the manufacturing method of the ultrasound examination device 14.
Fig. 6 is a schematic diagram showing the vicinity of the distal end of the ultrasound examination device 14 according to a modification example.

### MODES FOR CARRYING OUT THE INVENTION

Hereinafter, a preferred embodiment of the present invention will be described. Note that it goes without saying that the present embodiment is merely one embodiment of the present invention, and that the embodiment can be changed without departing from the gist of the present invention.

### [Catheter 10]

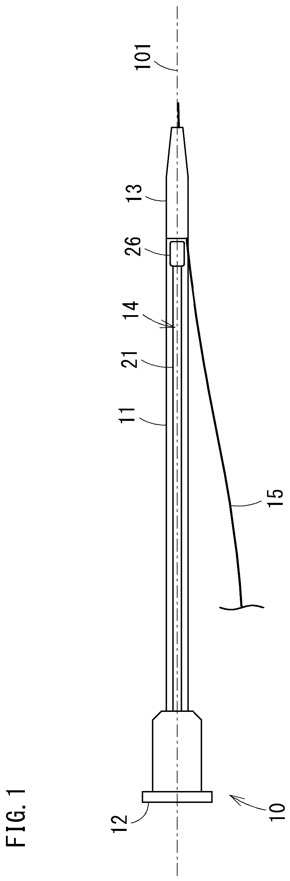

As shown in Fig. 1, a catheter 10 (an example of a catheter for intravascular ultrasound examination) includes an outer shaft 11, a hub 12, a distal end tip 13, and an ultrasound examination device 14 provided in the outer shaft 11. The catheter 10 is used as a medical instrument for inserting into a blood vessel and capturing an ultrasound image of the blood vessel.

The outer shaft 11 is a tube into whose internal space the ultrasound examination device 14 is rotatably inserted and through which liquid such as a physiological saline solution can flow. The outer shaft 11 is made of a circular tube made of medical stainless steel or a circular tube made of a synthetic resin, for example, and has flexibility to bend elastically in accordance with a curved shape of the blood vessel. Note that a material that transmits ultrasound waves is selected for a tip end side of the outer shaft 11. A tip end and a base end of the outer shaft 11 are each open. An outer diameter of the outer shaft 11 is set in accordance with an inner diameter of the blood vessel into which it is to be inserted, a coronary artery, for example, and is within a range of 0.6 to 1.3 mm, for example. An inner diameter of the outer shaft 11 is set in accordance with a size of the ultrasound examination device 14, and is within a range of 0.4 to 1.1 mm, for example. The outer diameter and the inner diameter of the outer shaft 11 are approximately uniform over an axial line direction 101 of the outer shaft 11. A length of the outer shaft 11 in the axial line direction 101 is set taking into consideration a length from a catheter insertion portion, such as a human limb, to an affected area or a length to a torque generating unit, and is within a range of 1000 to 1800 mm, for example.

The hub 12 is connected to a proximal end of the outer shaft 11. The hub 12 is a member having a cylindrical shape, and its internal space is continuous with an internal space of the outer shaft 11. The hub 12 is a portion that a practitioner holds to operate the catheter 10, and is also a portion that serves as a connector for connecting to an external device not shown. Although not shown in each drawing, the hub 12 is provided with a port for allowing liquid to flow, terminals for sending and receiving electrical signals, or the like.

The distal end tip 13 is connected to a distal end of the outer shaft 11. The distal end tip 13 is a member having a cylindrical shape whose outer diameter becomes thinner in a tapered manner toward a distal end, and its internal space is continuous with the internal space of the outer shaft 11. An opening into which a guide wire 15 is insertable is formed on a side surface of the distal end tip 13. The guide wire 15 is inserted from the side surface of the distal end tip 13 and is extended out from an opening at the distal end.

### [Ultrasound examination device 14]

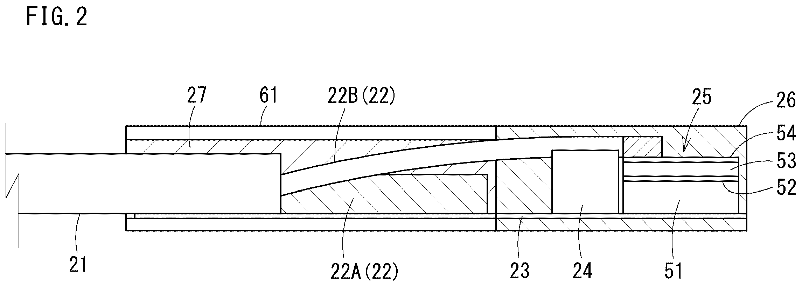

As shown in Figs. 1 and 2, the ultrasound examination device 14 has a shaft 21, a coaxial cable 22, a conductive thin film 23, an insulating support member 24, an ultrasound probe 25, a resin holder 26, a resin tube 61, and a conductive adhesive 27. The ultrasound examination device 14 outputs electrical signals from the ultrasound probe 25 when it is moved toward the proximal end of the outer shaft 11 while being rotated taking the axial line direction 101 of the shaft 21 as a center in the internal space of the outer shaft 11. Based on the electrical signals output from the ultrasound examination device 14, the external device not shown generates and displays an image of the blood vessel.

The shaft 21 is a tube into whose internal space the coaxial cable 22 is insertable and that can transmit, to a distal end, a rotational torque transmitted to a proximal end. The shaft 21 is configured by winding a wire material made of medical stainless steel into a double-coil shape, for example, and has flexibility to bend elastically in accordance with the curved shape of the blood vessel. Note that the shaft 21 may have a triple coil shape. When the shaft 21 has the triple coil shape, a first layer and a third layer are configured by coils having a same helical direction, and a second layer is configured by a coil having a different helical direction. An outer diameter of the shaft 21 is preferably as thin as possible, and is within a range of 0.35 to 1.0 mm, for example. The outer diameter of the shaft 21 is approximately uniform over the axial line direction 101 of the shaft 21. A length of the shaft 21 in the axial line direction 101 is set taking into consideration the length from the catheter insertion portion, such as the human limb, to the affected area or the length to the torque generating unit, and is within a range of 1000 to 1800 mm, for example.

In the coaxial cable 22, a conductive second lead wire 22B coated with an insulator extends linearly at a center, a first lead wire 22A is located around the second lead wire 22B, and a circumference of the first lead wire 22A is coated with an insulating material. The second lead wire 22B may be obtained by bundling a plurality of thin wires and coating it with an insulator, or may be a single wire. The first lead wire 22A is a plurality of thin wires arranged around an outer circumferential surface of the second lead wire 22B. The plurality of thin wires constituting the first lead wire 22A may extend linearly along the second lead wire, or the plurality may be twisted together. The first lead wire 22A shown in Fig. 2 becomes a single wire bundle by gathering portions extending out from a distal end portion of the shaft 21 toward a distal side to one side in a circumferential direction so as to align in parallel with the second lead wire 22B and twisting them together. Distal ends of the first lead wire 22A and the second lead wire 22B are extended out from the distal end of the shaft 21, and are each electrically connected to the ultrasound probe 25. Proximal ends of the first lead wire 22A and the second lead wire 22B are electrically connected to the external device not shown. Electrical signals are sent and received between the ultrasound probe 25 and the external device through the first lead wire 22A and the second lead wire 22B.

The first lead wire 22A is electrically connected to the ultrasound probe 25 through the conductive thin film 23. The conductive thin film 23 is a conductive member or a member having a thin film shape on whose surface a conductive member is laminated. The conductive thin film 23 has a rectangular shape in a plan view, a width dimension (dimension perpendicular to a sheet surface in Fig. 2) is equivalent to or smaller than a width of the ultrasound probe 25, a length dimension (dimension in a left-right direction in Fig. 2) is sufficiently longer than a length of the ultrasound probe 25, the width dimension is within a range of 0.2 to 0.5 mm, and the length dimension is within a range of 1.0 to 10 mm, for example. Furthermore, a thickness of the conductive thin film 23 (dimension in an up-down direction in Fig. 2) is sufficiently thinner than a thickness of the ultrasound probe 25 and an outer diameter of the coaxial cable 22, and is within a range of 10 to 200 µm for example. The conductive thin film 23 extends from the distal end portion of the shaft 21 toward the distal side, and contacts with a lower surface (an example of a first surface) of the ultrasound probe 25 to be electrically connected to it. The distal end of the first lead wire 22A contacts with the conductive thin film 23 between the distal end of the shaft 21 and the ultrasound probe 25 to be electrically connected to it.

The ultrasound probe 25 is located at a distal end of the conductive thin film 23. The ultrasound probe 25 has an outer shape of a flat plate, and piezoelectric ceramics generate ultrasound waves when an AC voltage is applied. It is preferable that the ultrasound probe 25 have small dimensions in order to make the ultrasound examination device 14 have a small diameter. In the present embodiment, the ultrasound probe 25 has an elongated outer shape in which a length dimension is longer than a width dimension, the width dimension is within a range of 0.3 to 0.5 mm, the length dimension is within a range of 0.6 to 1.0 mm, and a thickness is within a range of 0.2 to 0.4 mm, for example.

The ultrasound probe 25 is formed by laminating a backing layer 51, a first electrode layer 52, a piezoelectric ceramics sheet 53 (an example of a piezoelectric element sheet), and a second electrode layer 54 in order from a lower side. Note that in the ultrasound probe 25, an acoustic matching layer may be laminated on the second electrode layer 54, or other layers may be interposed, for example. The backing layer 51 is laminated to absorb sound, and has conductivity. The conductive thin film 23 contacts with the backing layer 51, and is electrically connected to it. As a result, the first lead wire 22A and the first electrode layer 52 are electrically connected. The distal end of the second lead wire 22B with its insulator coating being removed is adhered to a proximal end side of the second electrode layer 54 by a conductive adhesive on an upper surface (an example of a second surface) of the ultrasound probe 25, and is electrically connected to it.

The insulating support member 24 is located between the distal end of the first lead wire 22A and the ultrasound probe 25 and adjacent to the ultrasound probe 25. The insulating support member 24 is a member having an outer shape of a flat plate and is made of an insulating resin such as acrylic, for example, and a concave groove 24B to which the second lead wire 22B fits is formed on an upper surface 24A along the axial line direction 101. The second lead wire 22B is supported by the insulating support member 24 by being fitted to the concave groove 24B. It is preferable that a lowest position of a surface defining the concave groove 24B be almost at a same level as an upper surface of the ultrasound probe 25 or slightly below it. The surface defining the concave groove 24B may be inclined so as to become lower from a distal end toward a proximal end. It is preferable that a length of the insulating support body 24 along the concave groove 24B be within a range of 0.2 to 0.5 mm, because if it is too long, passing ability through the blood vessel is impaired.

The resin holder 26 contains and holds a portion belonging to the second lead wire 22B and extended out from the shaft 21, a portion of the conductive thin film 23 on a distal end side, the insulating support member 24, and the ultrasound probe 25. The resin holder 26 is made of an ultraviolet-curable resin, for example.

Resin tubes 61, 62 are molded products obtained by molding a synthetic resin into a tube shape. As for the synthetic resin, polyamide elastomer is given, for example. An inner diameter of the resin tube 62 is almost at a same level or slightly larger than an outer diameter of the resin tube 61.

As for the conductive adhesive 27, an epoxy resin containing silver, nickel, or the like is given, for example.

### [Manufacturing method of the ultrasound examination device 14]

The ultrasound examination device 14 is manufactured by the following manufacturing method, for example.

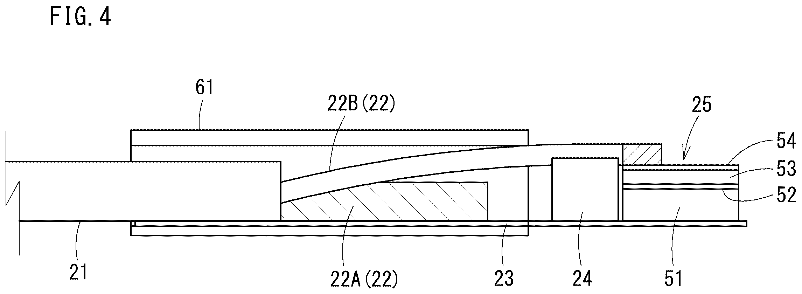

As shown in Fig. 4, the first lead wire 22A extended out from the distal end of the shaft 21 toward the distal side and the conductive thin film 23 extended out from the distal end portion of the shaft 21 toward the distal side are made to be located in an internal space of the resin tube 61. Furthermore, a portion of the second lead wire 22B other than a tip end portion is made to be located in the internal space of the resin tube 61. A proximal end of the resin tube 61 is located on a proximal side of the distal end of the shaft 21, and a distal end is located on the distal side of the distal end of the first lead wire 22A and on the proximal side of the distal end of the conductive thin film 23. At this time, the insulating support member 24 and the ultrasound probe 25 may be joined to the conductive thin film 23, or may not necessarily be arranged yet.

In a state where the shaft 21 and the first lead wire 22A are held in the internal space of the resin tube 61, the conductive adhesive 27 is filled into the internal space of the resin tube 61 from the distal end and is cured. With this, the conductive thin film 23 is fixed to the distal end of the shaft 21, and the conductive thin film 23 and the first lead wire 22A are adhered to be electrically connected.

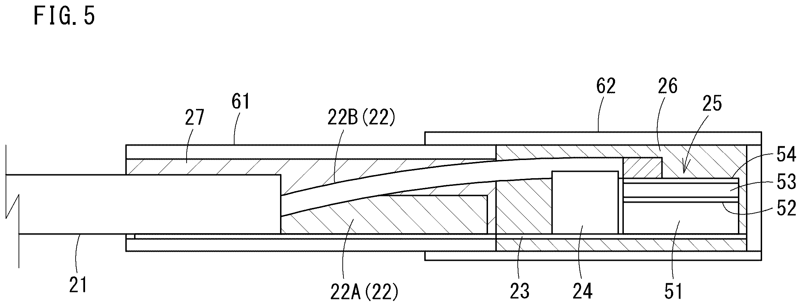

Next, as shown in Fig. 5, in a state where the insulating support member 24 and the ultrasound probe 25 are joined to the conductive thin film 23, and the second lead wire 22B is supported by the insulating support member 24 and is electrically connected to the upper surface of the ultrasound probe 25, they are inserted into an internal space of the resin tube 62. A proximal end of the resin tube 62 is located on the proximal side of the distal end of the first lead wire 22A, and a distal end is located on the distal side of the ultrasound probe 25.

The ultraviolet-curable resin is filled into the internal space of the resin tube 62 from the distal end and is cured. Thereafter, the resin tube 62 is removed. The cured ultraviolet-curable resin becomes the resin holder 26, which contains and holds the portion belonging to the second lead wire 22B and extended out from the resin tube 61, the portion of the conductive thin film 23 on the distal end side, the insulating support member 24, and the ultrasound probe 25.

### [Actions and effects of the present embodiment]

According to the ultrasound examination device 14 according to the present embodiment, since the first lead wire 22A is electrically connected to the ultrasound probe 25 through the conductive thin film 23, the ultrasound examination device 14 that has a small outer diameter dimension in a vicinity of the ultrasound probe 25 and is electrically stable is realized.

Furthermore, since the second lead wire 22B is supported by the insulating support member 24, a breakage of the second lead wire 22B near a connection point to the ultrasound probe 25 is suppressed.

Furthermore, since the conductive thin film 23 is connected to the lower surface of the ultrasound probe 25 and the second lead wire 22B is connected to the upper surface of the ultrasound probe 25, the conductive thin film 23 and the second lead wire 22B do not come close, and a short circuit between the conductive thin film 23 and the second lead wire 22B is suppressed.

Furthermore, since the first lead wire 22A and the second lead wire 22B form the coaxial cable, the internal space (inner diameter) of the shaft 21 can be made small.

Furthermore, since the resin holder 26 internally holds the portion belonging to the second lead wire 22B and extended out from the shaft 21, the conductive thin film 23, and the ultrasound probe 25, these members are insulated by the resin holder 26. With this, an individual insulation processing for each component is unnecessary. Furthermore, since the resin holder 26 coats these members integrally, freedom of an outer shape of the resin holder 26 is high, and it can be molded into a desired shape.

Furthermore, the resin tube 61 prevents the conductive adhesive 27 that is a relatively soft material from being partially peeled off, or the like. This effect is achieved especially when a load is applied to the ultrasound examination device 14 while being rotated, or the like.

### [Modification examples]

Note that although the second lead wire 22B is supported by the insulating support member 24 in the above-described embodiment, the insulating support member 24 may not necessarily be provided. Furthermore, the first lead wire 22A and the second lead wire 22B may not necessarily form the coaxial cable, and each may be a conducive wire independently coated by an insulator. Furthermore, the ultrasound probe 25 may be held by the resin holder 26 in an inclined manner with its upper surface inclined appropriately. For example, the upper surface of the ultrasound probe 25 may be inclined by thickening a thickness of the distal end of the conductive thin film 23.

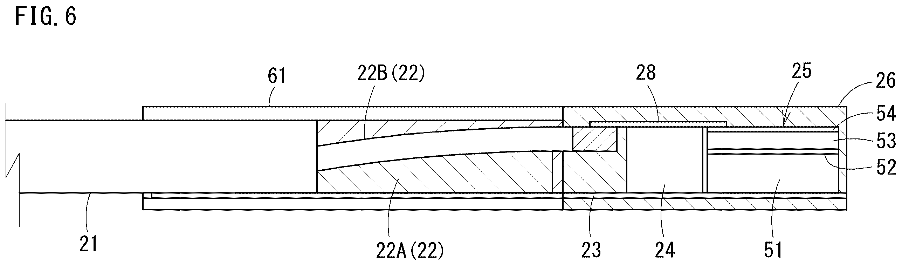

Furthermore, although in the above-described embodiment the distal end of the second lead wire 22B with its insulator coating being removed is adhered to the proximal end side of the second electrode layer 54 by the conductive adhesive on the upper surface of the ultrasound probe 25 and is electrically connected, it may be electrically connected to the ultrasound probe 25 through a conductive thin film. In Fig. 6, a conductive thin film 28 extends beyond a distal end and a proximal end of the insulating support member 24. The conductive thin film 28 is electrically connected to the distal end of the second lead wire 22B from which the insulation coating is removed. The conductive thin film 28 is laminated on a portion of the proximal end side of the second electrode layer 54 of the ultrasound probe 25, and is electrically connected to it. With this, the second lead wire 22B is electrically connected to the second electrode layer 54 of the ultrasound probe 25 through the conductive thin film 28. The conductive thin film 28 is a conductive member or a member having a thin film shape on whose surface a conductive member is laminated. The conductive thin film 28 is supported by the insulating support member 24. With this, an outer diameter dimension of the resin tube 61 can be made smaller than that of the above-described embodiment.

Furthermore, the manufacturing method of the ultrasound examination device 14 is not limited to the method shown in the embodiment. For example, without using the resin tubes 61, 62, in a state where the distal end portion of the shaft 21, the first lead wire 22A, the second lead wire 22B, the conductive thin film 23, the insulating support member 24, and the ultrasound probe 25 are held in an internal space of a single resin tube, an ultraviolet-curable resin may be filled to the internal space of the resin tube and may be cured. In this case, the resin holder 26 contains and holds the distal end portion of the shaft 21, portions belonging to the first lead wire 22A and the second lead wire 22B and extending out from the shaft 21, the conductive thin film 23, the insulating support member 24, and the ultrasound probe 25. Furthermore, the outer shape of the resin holder 26 may be formed in a desired shape by arranging the resin tube in a mold and filling the ultraviolet-curable resin or the like.

### DESCRIPTION OF REFERENCE CHARACTERS

10: catheter (catheter for intravascular ultrasound examination)
11: outer shaft
14: ultrasound examination device
21: shaft
22: coaxial cable
22A: first lead wire
22B: second lead wire
23, 28: conductive thin film
24: insulating support member
25: ultrasound probe
26: resin holder
51: backing layer
52: first electrode layer
53: piezoelectric ceramic sheet
54: second electrode layer

## Claims

1. An ultrasound examination device comprising:
a shaft;
a first lead wire and a second lead wire inserted into an internal space of the shaft and extended out from a distal end of the shaft;
a conductive thin film extending from the distal end of the shaft toward a distal side and electrically connected to the first lead wire;
an ultrasound probe located on the distal side of the distal end of the shaft and having a first electrode layer electrically connected to the conductive thin film and a second electrode layer electrically connected to the second lead wire; and
a resin holder configured to hold, at least, a portion belonging to the second lead wire and extended out from the shaft, the conductive thin film, and the ultrasound probe.

2. The ultrasound examination device according to claim 1, further comprising an insulating support member located between the distal end of the shaft and the ultrasound probe so as to be held by the resin holder, and configured to support the second lead wire.

3. The ultrasound examination device according to claim 1 or 2, wherein
the ultrasound probe has a flat plate shape in which a backing layer, the first electrode layer, a piezoelectric element sheet, and the second electrode layer are laminated,
the conductive thin film is connected to a first surface of the ultrasound probe on a side of the backing layer, and
the second lead wire is connected to a second surface on a side of the second electrode.

4. The ultrasound examination device according to claim 1 or 2, wherein the first lead wire and the second lead wire form a coaxial cable with the second lead wire located at a center.

5. The ultrasound examination device according to claim 1, wherein the resin holder is configured to internally hold the portion belonging to the second lead wire and extended out from the shaft, the conductive thin film, and the ultrasound probe.

6. A catheter for an intravascular ultrasound examination comprising:
the ultrasound examination device according to claim 1; and
an outer shaft into which the ultrasound examination device is inserted rotatably taking the shaft as an axis.
